Europäisches Patentamt

European Patent Office

Office européen des brevets

(19)

(11) Veröffentlichungsnummer: **0 037 883**
**B1**

(12) **EUROPÄISCHE PATENTSCHRIFT**

(45) Veröffentlichungstag der Patentschrift:
**23.03.83**

(21) Anmeldenummer: **81101047.9**

(22) Anmeldetag: **14.02.81**

(51) Int. Cl.³: **C 07 C 139/14**, C 07 C 139/04, C 07 C 143/02

(54) **Verfahren zur Abtrennung von Schwefelsäure aus dem bei der Umsetzung von Paraffinen mit Schwefeldioxid, Sauerstoff und Wasser in Gegenwart von UV-Licht anfallenden Sulfoxidationsaustrag.**

(30) Priorität: **10.04.80 DE 3013808**

(43) Veröffentlichungstag der Anmeldung:
**21.10.81 Patentblatt 81/42**

(45) Bekanntmachung des Hinweises auf die Patenterteilung:
**23.03.83 Patentblatt 83/12**

(84) Benannte Vertragsstaaten:
**BE CH FR GB IT LI NL SE**

(56) Entgegenhaltungen:
**DE-A-2 014 783**
**DE-A-2 855 849**

(73) Patentinhaber: **CHEMISCHE WERKE HÜLS AG,
Postfach 1320, D-4370 Marl 1 (DE)**

(72) Erfinder: **Springmann, Hermann, Dr., Am Mühlenberg 2,
D-4358 Haltern (DE)**
Erfinder: **Borchers, Karl, Bramgaustrasse 14,
D-4280 Borken 1 (DE)**

0 037 883

## Verfahren zur Abtrennung von Schwefelsäure aus dem bei der Umsetzung von Paraffinen mit Schwefeldioxid, Sauerstoff und Wasser in Gegenwart von UV-Licht anfallenden Sulfoxidationsaustrag

Unter der Sulfoxidation versteht man bekanntermaßen die gemeinsame Einwirkung von Schwefeldioxid und Sauerstoff auf gegebenenfalls funktionelle Gruppen tragende n-Paraffine oder Cycloparaffine, wobei Paraffinsulfonsäuren entstehen.

Die praktisch durchführbaren Varianten der Reaktion unterscheiden sich in erster Linie durch die Art der Anregung der Sulfoxidation. So kennt man das UV-Licht-, das Ozon-, das $\gamma$-Strahlen-Verfahren und Verfahren durch Zugabe von Peroxidverbindungen. Außerdem gibt es das Essigsäureanhydrid-, das Licht-Wasser- und das Chlor-Verfahren [Chemie in unserer Zeit, 13 (1979), Seite 157 ff.].

Bei der Sulfoxidation nach dem Licht-Wasser-Verfahren, d. h. bei der Umsetzung von Paraffinen mit Schwefeldioxid, Sauerstoff und Wasser in Gegenwart von UV-Licht, entsteht neben den erwünschten Paraffinsulfonsäuren auch Schwefelsäure, die im Laufe der Aufarbeitung des Sulfoxidationsaustrags abgetrennt werden muß. Unter Sulfoxidationsaustrag versteht man dabei — auch im Rahmen der vorliegenden Erfindung — den bereits entgasten und vom größten Teil des nicht umgesetzten Paraffins befreiten Reaktionsaustrag.

Zur Abtrennung der Schwefelsäure geht man im allgemeinen so vor, daß man den Sulfoxidationsaustrag mit einem geeigneten organischen Lösemittel behandelt, um eine Entmischung in eine organische Phase, welche die Gesamtheit der Paraffinsulfonsäuren enthält, und in eine wäßrige Phase, welche die Schwefelsäure soweit wie möglich in Form einer im allgemeinen 15- bis 25%igen Lösung enthält, herbeizuführen. Die beiden Phasen werden dann getrennt und die organische Phase auf die Isolierung der Paraffinsulfonsäuren bzw. ihrer Salze hin weiter aufgearbeitet.

So ist aus der deutschen Patentanmeldung mit dem Aktenzeichen F 3718. 120, 23/01 bereits bekannt, Benzol, Chlorbenzol, Cyclohexan, Tetrachlorkohlenstoff, Chloroform, Methylenchlorid, 1,2-Dichlorethan, Tetrahydrofuran und — aus der Gruppe der Alkohole — Butanol als organische Lösemittel einzusetzen.

Gemäß dem Verfahren der französischen Patentschrift 1 478 530 können als organische Lösemittel ganz allgemein sauerstoffhaltige Verbindungen, wie Alkohole, Ether und Ketone, verwendet werden. Aus der Gruppe der Alkohole ist der Amylalkohol besonders hervorgehoben.

Wie nun aus der deutschen Offenlegungsschrift 2 745 691, in der Ether, Ketone, Ester und (cyclo)-aliphatische Ketoester als organische Lösemittel beschrieben sind, hervorgeht (1. c. Seiten 8 und 9), können beim Einsatz von Alkoholen mit mindestens 5 Kohlenstoffatomen (DE-OS 2 139 477) bei der kontinuierlichen Durchführung des Verfahrens bestimmte Schwierigkeiten auftreten, weil der eingesetzte Alkohol nach seiner Rückgewinnung nicht ausreichend von nicht sulfonierten Paraffinen befreit ist.

Aufgabe der vorliegenden Erfindung war es daher, das Licht-Wasser-Verfahren so zu gestalten, daß es einmal möglich ist, auch Alkohole mit mindestens 5 Kohlenstoffatomen bei der Abtrennung der Schwefelsäure einzusetzen, ohne daß bei der Rückgewinnung der Einsatzstoffe aufwendige Trennoperationen erforderlich sind, und daß zum anderen gleichzeitig das Trennverhalten (Verweilzeit im Abscheider und Abscheidungsgrad der vorhandenen Schwefelsäure) weiter verbessert wird.

Diese Aufgabe wurde überraschenderweise durch die in den Patentansprüchen beschriebenen Maßnahmen gelöst. Überraschend deswegen, weil es angesichts der zitierten Ausführungen in der DE-OS 2 745 691 keineswegs zu erwarten war, daß durch den Zusatz von bestimmten Mengen Paraffin das gute Trennverhalten von Alkoholen mit mindestens 5 Kohlenstoffatomen noch verbessert werden kann.

Im allgemeinen geht man bei der Durchführung des erfindungsgemäßen Verfahrens so vor, daß man den Sulfoxidationsaustrag zunächst bei einer Temperatur von 10 bis 60° C, vorzugsweise bei 20 bis 45° C, eine bestimmte Zeit lang, die durch orientierende Versuche leicht ermittelt werden kann, mit der 0,25- bis 2,5fachen, vorzugsweise 0,5- bis 1,75fachen Gewichtsmenge der Mischung aus a) Alkohol und b) Parafin behandelt.

In der Mischung, bestehend aus a) und b), sind dabei 40 bis 95, vorzugsweise 70 bis 85 Gewichtsprozent Alkohol und 5 bis 60, vorzugsweise 15 bis 30 Gewichtsprozent Paraffin.

Die Alkohole, die beim erfindungsgemäßen Verfahren eingesetzt werden können, besitzen 5 bis 12, vorzugsweise 6 bis 9 Kohlenstoffatome. Typische Vertreter sind Pentanol-1, Hexanol-1, Heptanol-1, Octanol-1, Nonanol-1, Decanol-1, Dodecanol-1, 2-Ethylbutanol-1, 2-Methylpentanol, 2-Ethylhexanol-1, 2,6-Dimethyl-4-heptanol, 3-Ethylhexanol-1,2,7-Dimethyloctanol, Octanol-2 und Cyclohexanol. Selbstverständlich können auch Alkoholgemische eingesetzt werden. Bevorzugt werden beim erfindungsgemäßen Verfahren 2-Ethylhexanol-1, Heptanol-1, Octanol-1 und Nonanol-1 verwendet.

Als Paraffine werden solche mit 7 bis 30, bevorzugt solche mit 10 bis 20 Kohlenstoffatomen eingesetzt.

In der Regel sind die für die Phasentrennung eingesetzten Paraffine mit den Ausgangsparaffinen identisch oder weitgehend identisch. Eine nur weitgehende Identität kann z. B. daher rühren, daß sich beim Einsatz von Paraffinschnitten bei der Sulfoxidation dessen Zusammensetzung im Laufe des Gesamtsulfoxidationsprozesses ändert.

Im Anschluß an die Behandlung, d. h. an die gute Durchmischung des Sulfoxidationsaustrags mit

2

der Mischung aus Alkohol und Paraffin, die in jedem handelsüblichen Mischaggregat, wie z. B. einem Rührkessel, durchgeführt werden kann, wartet man die Ausbildung der beiden Phasen ab und trennt diese dann. Die Trennung kann sowohl kontinuierlich als auch diskontinuierlich durchgeführt werden.

Die Aufarbeitung der oberen organischen Phase kann auf verschiedene Art und Weise erfolgen. Sie richtet sich in erster Linie danach, ob man die freien Paraffinsulfonsäuren oder die Paraffinsulfonate, vorzugsweise Alkalisulfonate, erhalten will.

Um die freien Paraffinsulfonsäuren zu erhalten, entfernt man den Alkohol und das Paraffin, z. B. durch azeotrope Destillation bei einem Druck, der höchstens gleich dem Atmosphärendruck ist. Zu diesem Zweck steuert man die Wassermenge in der alkoholischen/paraffinischen Lösung der Paraffinsulfonsäuren so, daß die Gesamtmenge an Alkohol und Paraffin im azeotropen Gemisch mit dem Wasser abdestilliert werden kann. Die Paraffinsulfonsäuren werden im Bodenteil der Kolonne in Form einer Flüssigkeit gewonnen, die gegebenenfalls zur Überführung in Sulfonate neutralisiert werden kann.

Zur Abtrennung der Paraffinsulfonsäuren in Form von Sulfonaten geht man so vor, daß man die organische Phase vor der Abtrennung von Alkohol und Paraffin mit einer basischen Verbindung behandelt. Geeignete basische Verbindungen sind Hydroxide und Carbonate der Alkalimetalle, vorzugsweise des Natriums und Kaliums, sowie Oxide, Hydroxide und Carbonate der Erdalkalimetalle, vorzugsweise des Calciums. Natriumhydroxid ist als basische Verbindung und damit als Neutralisationsmittel besonders bevorzugt.

Die basischen Verbindungen können in fester Form oder als wäßrige Lösungen und in Mengenverhältnissen, die mindestens für die Neutralisation der Gesamtmenge der Paraffinsulfonsäuren ausreichen, eingesetzt werden. Vorzugsweise arbeitet man mit einer Menge, die etwas größer als die stöchiometrisch erforderliche Menge ist, z. B. bis zu 1,2 Val der basischen Verbindung pro Val Paraffinsulfonsäure.

Aus der so behandelten organischen Phase werden die paraffinsulfonsauren Salze sodann in bekannter Weise, z. B. über einen Dünnschichtverdampfer, von dem Extraktionsmittel, d. h. der Mischung aus Alkohol und Paraffin und dem Wasser befreit. Die Paraffinsulfonate werden dabei als Schmelze aus dem Sumpf des Verdampfers abgezogen. Als Kopfprodukt wird ein Gemisch, bestehend aus Alkohol, Paraffin und Wasser, erhalten, das sich in eine organische und eine wäßrige Phase auftrennt.

Die organische Phase, bestehend aus Alkohol und Paraffin, braucht anschließend, gegebenenfalls nur so weit von Paraffin befreit zu werden, daß eine Mischung in der Zusammensetzung zurückbleibt, die einem Sulfoxidationsaustrag zwecks Phasentrennung wieder zugesetzt werden kann. Das abgetrennte Paraffin geht zweckmäßigerweise wieder in den Sulfoxidationsprozeß zurück.

Die nach dem vorliegenden Verfahren gewonnenen bzw. isolierten Produkte, freie Paraffinsulfonsäuren bzw. deren Alkali- oder Erdalkalisalze, dienen vornehmlich zur Herstellung von Detergentienformulierungen.

Alle Prozentangaben, auch in den nachfolgenden Beispielen und Vergleichsbeispielen, die das erfindungsgemäße Verfahren erläutern, sind, sofern nicht anders angegeben, Gewichtsprozente.

In allen Beispielen und Vergleichsbeispielen wurde von einem Sulfoxidationsaustrag der folgenden prozentualen Zusammensetzung ausgegangen:

| | |
|---|---|
| Paraffinsulfonsäuren: | 22,0 |
| Paraffin: | 30,0 |
| Wasser: | 39,6 |
| Schwefelsäure: | 7,4 |

Das für die Sulfoxidation und als Komponente b) verwendete Paraffingemisch hatte folgende Zusammensetzung:

| C-Zahl | Gew.-% |
|---|---|
| $C_{12}$ | 1,07 |
| $C_{13}$ | 9,49 |
| $C_{14}$ | 27,8 |
| $C_{15}$ | 19,8 |
| $C_{17}$ | 10,2 |
| $C_{18}$ | 3,04 |
| $C_{19}$ | 0,5 |

3

### Beispiele 1 bis 7 und Vergleichsbeispiel A

Jeweils 200 g des Sulfoxidationsaustrags der genannten Zusammensetzung wurden mit den in Spalte 2 der Tabelle 1 angegebenen Mengen an 2-Ethylhexanol-(1) (Vergleichsbeispiel A) bzw. einer definierten Mischung aus 2-Ethylhexanol-(1) (a) und Paraffin (b) versetzt. Bei einer Temperatur von 25°C wurde das Gemisch gut verrührt und zur Trennung in ein Absitzgefäß gegeben. Nach den in Spalte 4 der Tabelle 1 angegebenen Verweilzeiten wurde die wäßrige Phase zum ersten Mal (I), nach 30 Minuten zum zweiten Mal (II) und nach 24 Stunden zum dritten Mal (III) abgetrennt. Den Abscheidungsgrad der Schwefelsäure, bezogen auf die im Sulfoxidationsaustrag vorhandene Schwefelsäure, zeigen die diesbezüglichen Spalten der Tabelle 1.

### Beispiele 8 bis 14 und Vergleichsbeispiel B

Die Beispiele 1 bis 7 und Vergleichsbeispiel A wurden mit dem Unterschied wiederholt, daß die Vermischung des Sulfoxidationsaustrags mit a) bzw. a) und b) nicht bei 25°C, sondern bei 40°C durchgeführt wurde. Die Ergebnisse zeigt Tabelle 2.

### Vergleichsbeispiel C

200 g des angegebenen Sulfoxidationsaustrags wurden mit 250 g 2-Ethylhexanol-(1) versetzt und bei 25°C gut vermischt. Nach 30 Minuten konnten 84,6 g untere wäßrige Phase mit einem Gehalt von 16,3% Schwefelsäure abgetrennt werden. Daraus errechnet sich ein Abscheidungsgrad der Schwefelsäure von 93,2%. Dieser Wert ist mit der 95,7%igen Abscheidung des Beispiels 3 in Tabelle 1 zu vergleichen.

### Vergleichsbeispiel D

Vergleichsbeispiel C wurde mit dem Unterschied wiederholt, daß die Behandlung des Sulfoxidationsaustrags bei 40°C durchgeführt wurde. Nach 30 Minuten wurden 87,1 g untere wäßrige Phase mit einem Gehalt von 16,3% Schwefelsäure abgetrennt. Daraus ergibt sich eine 95,9%ige Abtrennung der Schwefelsäure. Dieser Wert ist mit der 99,6%igen Abscheidung des Beispiels 10 in Tabelle 2 zu vergleichen.

Tabelle 1

| Beisp. bzw. Vgl.-Bsp. | Menge a bzw. a+b [g] | Anteil b in a+b [Gew.-%] | I Verweilzeit [min] | Menge der abgetrennten unteren Phase [g] | II Verweilzeit [min] | Menge der abgetrennten unteren Phase [g] | III Verweilzeit [h] | Menge der abgetrennten unteren Phase [g] | Gesamtmenge der unteren Phasen $[\Sigma \text{I–III}]$ [g] | Anteil $H_2SO_4$ in $\Sigma$ I–III [Gew.-%] | Abgetrennte $H_2SO_4$, bezogen auf die Menge $H_2SO_4$ im Austrag [Gew.-%] | | |
|---|---|---|---|---|---|---|---|---|---|---|---|---|---|
| | | | | | | | | | | | I | I + II | I + II + III |
| A | 200 | 0 | 12 | 64,7 | 30 | 10,5 | 24 | 4 | 79,2 | 16,9 | 77,3 | 85,9 | 90,4 |
| 1 | 210,5 | 5 | 10 | 75,7 | 30 | 6,6 | 24 | 2,5 | 84,8 | 16,5 | 84,4 | 91,7 | 94,5 |
| 2 | 222,2 | 10 | 9 | 77,1 | 30 | 5,9 | 24 | 2,6 | 85,6 | 16,8 | 87,5 | 94,2 | 97,2 |
| 3 | 250 | 20 | 5 | 80,2 | 30 | 4,1 | 24 | 1,5 | 85,8 | 16,8 | 91 | 95,7 | 97,4 |
| 4 | 285,7 | 30 | 6 | 74,2 | 30 | 7,8 | 24 | 3,1 | 85,1 | 17 | 85,2 | 95,3 | 97,8 |
| 5 | 333,3 | 40 | 6 | 78,2 | 30 | 5,4 | 24 | 2,5 | 86,1 | 17,1 | 90,4 | 96,6 | 99,5 |
| 6 | 400 | 50 | 5,5 | 75,9 | 30 | 7,7 | 24 | 2,8 | 86,4 | 16,9 | 86,7 | 95,5 | 98,7 |
| 7 | 500 | 60 | 6 | 76,8 | 30 | 7,5 | 24 | 3 | 87,3 | 16,8 | 87,2 | 95,7 | 99,1 |

Tabelle 2

| Beisp. bzw. Vgl.-Bsp. | Menge a bzw. a+b [g] | Anteil b in a+b [Gew.-%] | I Verweil-zeit [min] | Menge der abgetrenn-ten unteren Phase [g] | II Verweil-zeit [min] | Menge der abgetrenn-ten unteren Phase [g] | III Verweil-zeit [h] | Menge der abgetrenn-ten unteren Phase [g] | Gesamt-menge der unteren Phasen [$\Sigma$ I−III] [g] | Anteil H$_2$SO$_4$ in $\Sigma$ I−III [Gew.-%] | Abgetrennte H$_2$SO$_4$, bezogen auf die Menge H$_2$SO$_4$ im Austrag [Gew.-%] I | I + II | I + II + III |
|---|---|---|---|---|---|---|---|---|---|---|---|---|---|
| B | 200 | 0 | 4 | 78,3 | 30 | 7,6 | 24 | 1,2 | 87,1 | 16,2 | 85,7 | 94 | 95,3 |
| 8 | 210,5 | 5 | 4,3 | 83 | 30 | 4,6 | 24 | 1,7 | 89,3 | 16,4 | 92 | 97 | 99,0 |
| 9 | 222,2 | 10 | 3,9 | 79,8 | 30 | 7,3 | 24 | 1,4 | 88,5 | 16,3 | 87,9 | 95,9 | 97,5 |
| 10 | 250 | 20 | 2,9 | 84,8 | 30 | 5,6 | 24 | 0,7 | 91,1 | 16,3 | 93,4 | 99,6 | 100,0 |
| 11 | 285,7 | 30 | 4,2 | 84,4 | 30 | 4,4 | 24 | 1,1 | 89,9 | 16,2 | 92,4 | 97,2 | 98,4 |
| 12 | 333,3 | 40 | 2,8 | 82,8 | 30 | 6,8 | 24 | 1,1 | 90,7 | 16,1 | 90,1 | 97,5 | 98,7 |
| 13 | 400 | 50 | 2,8 | 82,5 | 30 | 8,2 | 24 | 0,8 | 91,5 | 15,9 | 88,6 | 97,4 | 98,3 |
| 14 | 500 | 60 | 3,3 | 79,1 | 30 | 10,3 | 24 | 1,3 | 90,7 | 16,0 | 85,5 | 96,6 | 98,1 |

**0 037 883**

## Patentansprüche

1. Verfahren zur Abtrennung von Schwefelsäure aus dem bei der Umsetzung von Paraffinen mit Schwefeldioxid, Sauerstoff und Wasser in Gegenwart von ultraviolettem Licht anfallenden Sulfoxidationsaustrag mit Hilfe eines Alkohols, der wenigstens 5 Kohlenstoffatome enthält, dadurch gekennzeichnet, daß man den Sulfoxidationsaustrag mit der 0,25- bis 2,5fachen Gewichtsmenge einer Mischung aus

a)   40 bis 95 Gewichtsprozent eines Alkohols mit 5 bis 12 Kohlenstoffatomen und
b)   5 bis 60 Gewichtsprozent eines Paraffins mit 7 bis 30 Kohlenstoffatomen

behandelt und die wäßrige Phase abzieht.

2. Verfahren nach Anspruch 1, dadurch gekennzeichnet, daß die Mischung aus

a)   70 bis 85 Gewichtsprozent Alkohol und
b)   15 bis 30 Gewichtsprozent Paraffin

besteht.

3. Verfahren nach Anspruch oder den Ansprüchen 1 und 2, dadurch gekennzeichnet, daß man Alkohole mit 6 bis 9 und Paraffine mit 10 bis 20 Kohlenstoffatomen einsetzt.

## Claims

1. A process for separating sulphuric acid from the sulphoxidation product occuring in the reaction of a paraffin with sulphur dioxide, oxygen and water in the presence of ultraviolet light, using an alcohol containing at least 5 carbon atoms in the separation, characterised in that the sulphoxidation product is treated with from 0.25 to 2.5 times its weight of a mixture of

a)   40 to 95% by weight of an alcohol of 5 to 12 carbon atoms and
b)   5 to 60% by weight of a paraffin of 7 to 30 carbon atoms,

and the aqueous phase is separated off.

2. A process according to claim 1, characterised in that the mixture consists of

a)   70 to 85% by weight of the alcohol, and
b)   15 to 30% by weight of the paraffin.

3. A process according to claim 1 or to claims 1 and 2, characterised in that an alcohol of 6 to 9 carbon atoms and a paraffin of 10 to 20 carbon atoms is used.

## Revendications

1. Procédé pour séparer, à l'aide d'un alcool renfermant au moins 5 atomes de carbone, l'acide sulfurique du produit de sulfoxydation obtenu en faisant réagir des paraffines sur de l'anhydride sulfureux, de l'oxygène et de l'eau en présence de lumière ultra-violette, ledit procédé étant caractérisé par le fait qu'on traite le produit de sulfoxydation par une quantité égale en poids à 0,25 – 2,5 fois celle d'un mélange constitué par

a)   40 à 95% en poids d'un alcool comportant de 5 à 12 atomes de carbone et par
b)   5 à 60% en poids d'une paraffine comportant de 7 à 30 atomes de carbone

et qu'on soutire la phase aqueuse.

2. Procédé selon la revendication 1, caractérisé par le fait que le mélange est constitué par

a)   70 à 85% en poids d'alcool et par
b)   15 à 30% en poids de paraffine.

3. Procédé selon la revendication 1 ou les revendications 1 et 2, caractérisé par le fait qu'on utilise des alcools comportant de 6 à 9 atomes de carbone et de paraffines comportant de 10 à 20 atomes de carbone.